# EUROPEAN PATENT APPLICATION

(11) **EP 4 751 741 A1**
(43) Date of publication of application: **03.06.2026**
(21) Application number: 25214485.2
(22) Date of filing: 09.11.2025
(51) Int. Cl.: A61L 9/20, A47G 1/00, F24F 8/22

(54) **COMPACT DEVICE FOR REFLECTING IMAGES AND DECONTAMINATING THE AIR, WITH PROTECTION THAT ENABLES CONTINUOUS HUMAN PRESENCE AND A SYSTEM FOR DETECTING AND WARNING OF ANY FAILURE OF THAT PROTECTION**

(30) Priority: 27.11.2024 ES 202530372
(71) Applicant: Garcia-Gallardo Sanz, Prospero, 09004 Burgos (ES)
(72) Inventor: Garcia-Gallardo Sanz, Prospero, 09004 Burgos (ES)

(57) **Abstract**

Compact device arranged to reflect images and decontaminate the air, with protection enabling continuous human presence and a system for detecting and warning of failure of said protection.

Air decontamination by ultraviolet radiation requires the emitter to be located within a radiation-opaque housing, which protects persons from the radiation, such that safe use is only possible if the housing is maintained in good condition. Since the air treatment capacity depends on the volume of air contained inside the radiation-opaque housing, it is difficult to obtain the necessary space in existing buildings in use. The proposed device makes it possible to obtain the space required to decontaminate air by ultraviolet radiation from spaces in existing buildings intended for image reflecting systems, such as mirrors, and can provide information on the condition of the housing, thereby ensuring safe use with respect to ultraviolet radiation. Fig. 6.

## Description

### FIELD OF THE INVENTION

The present invention relates to the field of fixed building installations, more particularly installations in premises equipped with sanitary appliances, which are intended for personal hygiene and the disposal of by-products of the human body.

The object of the invention is a compact device arranged to reflect images and to decontaminate the air by applying ultraviolet radiation, this combination providing a technical advantage.

### BACKGROUND OF THE INVENTION

Bathrooms, lavatories or service rooms are premises within buildings characterised by comprising appliances referred to as sanitary appliances, the most common being a toilet, urinal, bidet, shower, bathtub or washbasin. These appliances enable the disposal of faeces, urine, bleeding, sweat and acquired bodily dirt, etc., with the consequent emission, during the process, of contaminant aerosols of a chemical, biological and odorous nature, affecting the health and comfort of users; for this reason, this is a highly regulated aspect.

The removal of contaminant aerosols in such premises is necessary and is usually required by regulations-in Spain, for example, the CTE, RITE, RD 486/1997, etc.-and is carried out by directly opening windows to the outside, by means of natural air-extraction ducts of the shunt type, or by networks of mechanical extraction ducts.

It is evident that, with the first two systems, the result is variable and depends on external environmental conditions; in fact, shunt-type systems do not operate if there is no wind. Moreover, both systems entail an energy cost to condition the temperature of the indoor environment, or otherwise a loss of comfort in these premises.

Mechanical extraction is a more stable system and can recover part of the lost energy, but it requires complex duct networks and bulky equipment, with space requirements such that, if they were not provided for in the building's initial design, they may be difficult to implement and, in many cases, impossible, since they would alter the functionality, uses and activities existing in the building. Nevertheless, duct systems, whether for natural or mechanical extraction, present serious problems with internal cleaning, and this worsens as the building becomes older, in some cases leading to an increase in indoor contamination as a result rather than the opposite; this is one of the symptoms of the so-called "sick building".

As a recent alternative, certain regulations such as "ASHRAE Standard 241, Control of Infectious Aerosols" allow the use of indoor air recirculators as a complement to the foregoing systems, acknowledging that they may operate on the basis of one technology or several combined, whether of a mechanical, chemical or energy-based type. By way of example, mechanical technology includes HEPA filtration of biological agents, chemical technology includes odour adsorption by means of activated carbon, and energy-based technology includes application of ultraviolet radiation to the air. Each system has its advantages and disadvantages, whether due to limited effectiveness only against biological agents and not chemical agents, due to requirements for frequent maintenance, due to noise, due to energy consumption, or due to a significant space requirement, as is the case with ultraviolet radiation.

In addition, the number of sanitary appliances required in a building, their clear distances free of obstacles, the floor area of the premises in which they are located, etc., depend on the number of users and the intended activity, all of which is usually regulated by multiple overlapping regulations, of an urban-planning, construction, accessibility, gender-equality, activities-related and labour type. Consequently, the introduction of a new device for removing contaminant aerosols in premises equipped with sanitary appliances is also affected by these constraints.

According to the foregoing, the introduction of a new device in such premises, particularly when they are already in use, may entail a modification of the spatial configuration of the premises and, as a result of the application of the regulations, a possible reduction in the permitted number of users or in the capacity of the activity carried out in the building.

### EXPLANATION OF THE INVENTION

In premises equipped with sanitary appliances, intended for personal hygiene and the disposal of by-products of the human body, contaminant aerosols of a chemical, biological and odorous nature are generated, affecting the health and comfort of users. For this reason, such premises are ones in which the installation of an air decontaminator by ultraviolet radiation is particularly useful; however, it is often not possible to install such a device, since its effectiveness and energy efficiency are highly dependent on the available space, which is not present in many of the aforementioned premises. On the other hand, it is common for such premises to include a mirror, the shape and location of which are favourable for obtaining the necessary space, it being understood that the mirror remains useful and necessary.

The object of the invention is a compact device arranged to reflect images and decontaminate the air (1) by applying ultraviolet radiation, thereby providing a TECHNICAL ADVANTAGE:
Replacing the existing mirror simplifies the installation of an ultraviolet radiation emitting system (2) arranged to decontaminate the air in premises equipped with sanitary appliances, where it is particularly useful, and may even make such installation possible where it previously was not due to lack of space, which is obtained simply by removing the existing mirror.

In addition, the image reflecting system (5) may be more fragile and sensitive to impacts or abrupt movements, i.e. it breaks upon impact with an amount of energy that does not imply breakage of the radiation-opaque housing (3), such that its condition provides rapid visual information regarding possible damage to the radiation-opaque housing (3) forming the radiation chamber, which is a key element for user safety, as explained further below.

### Description and operation

Ultraviolet radiation is effective in eliminating many chemical, biological and odorous aerosols, but it may also be harmful to persons and other living beings; therefore, it is applied separated from them within a radiation chamber, which is formed by a radiation-opaque housing (3) inside which an ultraviolet radiation emitting system (2) is arranged. Air is introduced into and/or extracted from the radiation chamber by means of an air propulsion system (6)-a fan, a compressor, etc. The openings in the housing (4) for the inlet and/or outlet of air into/from the chamber must comprise devices that allow the passage of air and prevent the passage of radiation. Various systems exist for this purpose, for example broken/baffled ducts, parallel plates with non-aligned orifices, or a granular bed; see Patent Publication No. ES2907199 (2024).

Since the effectiveness of the treatment depends on the energy applied per surface area inside the chamber and on the time the air remains within it, it follows that the larger the chamber, the longer the air remains inside it for the same flow rate, and the greater the volume of air that can be treated with the same emitter in the same time. Consequently, the available space is decisive for the power required for the emitter and for the energy consumption of the device.

The proposed invention makes it possible to obtain suitable space by replacing an object having a favourable shape and which is usually present in premises equipped with sanitary appliances, namely a mirror. Since it is a predominantly surface-developing object mounted against a vertical wall above washbasins, it provides the possibility of forming a flattened radiation chamber, i.e. a chamber having a height dimension that is much smaller compared to its length or width. Air flows inside the chamber between the larger faces, i.e. in a direction perpendicular to the height. One of the larger faces is mounted to a wall, and the other comprises an image reflecting system (5); the term "mirror"-silvered glass on its rear side-is avoided in view of the multiple possibilities currently available for performing that function.

This chamber shape is particularly suitable for obtaining a uniform distribution of radiation energy. Since the radiation flux depends inversely on the distance from the emitter, air closer to the emitter receives a greater amount of energy than air further away, so that the received treatment may have differing effectiveness. To mitigate this effect, inward-facing areas coated with a material that reflects part of the radiation (7) may be provided. It is also effective to take advantage of the predominantly surface-developing configuration of the chamber to distribute the ultraviolet radiation emitting system (2) into multiple sources such that the air layer thicknesses are similar. A similar treatment is thus provided throughout the entire air volume, also contributing to energy efficiency.

In order to improve the quality of the treated air and user comfort, the device may comprise an air particle filtration system (8), an air humidity modifying system (9), an air temperature modifying system (10), a premises illumination system (11), a switch-on system actuated by detection of the presence of users in the premises (12), a switch-on system actuated by a timer (13), as well as a data logging system for environmental parameters (14), a data logging system for operating parameters (15) and a user information system arranged to provide users with information regarding operation (16).

### BRIEF DESCRIPTION OF THE DRAWINGS

To complement the description being provided and in order to facilitate a better understanding of the features of the invention, a set of drawings is included as an integral part of said description, in which, by way of illustration and not of limitation, the following is shown:

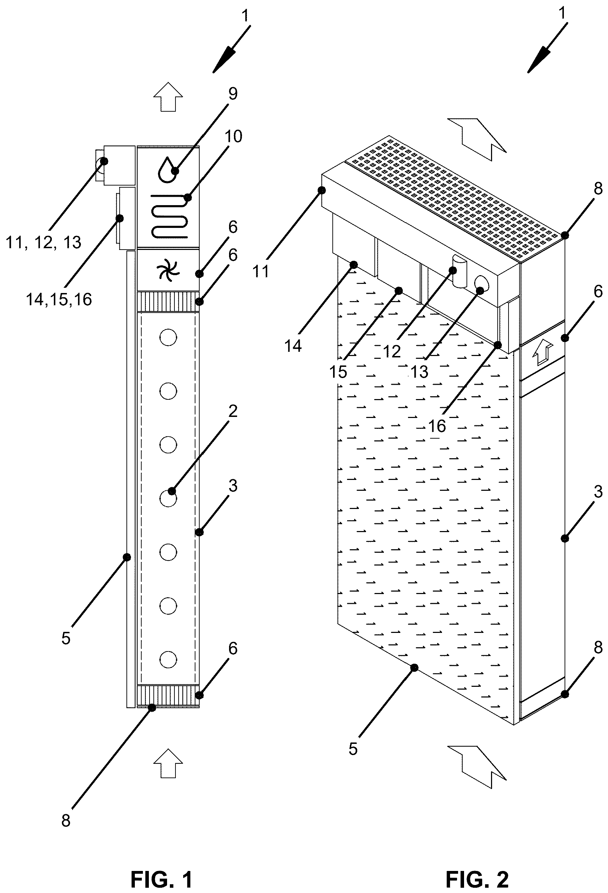

- Figure 1.: Shows a sectional side view of the device of the invention.
- Figure 2.: Shows an axonometric view of the device of the invention.

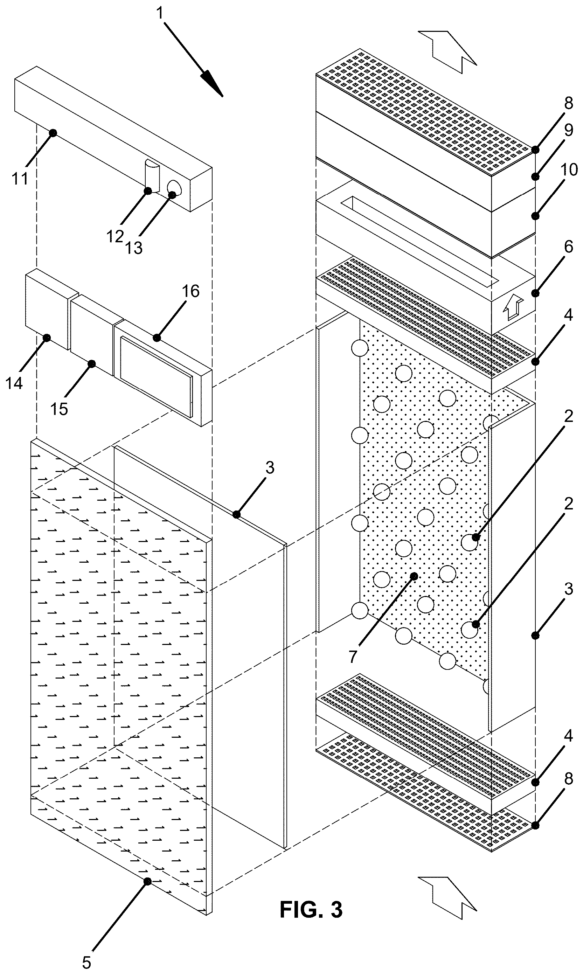

- Figure 3.: Shows an axonometric view with components displaced to facilitate understanding of the device of the invention.
- Figure 4.: Shows a sectional side view of a preferred embodiment of the device of the invention.
- Figure 5.: Shows an axonometric view of a preferred embodiment of the device of the invention.
- Figure 6.: Shows an axonometric view of a preferred embodiment with components displaced to facilitate understanding of the device of the invention.
- Figure 7.: Shows a small-size lavatory that removes its aerosols by means of a window: side view in 7.a), front view 7.b) and plan view 7.c).
- Figure 8.: Shows a small-size lavatory that removes its aerosols by means of a compact device arranged to reflect images and decontaminate the air in premises equipped with sanitary appliances: side view in 8.a), front view 8.b) and plan view 8.c).

In the figures, the unfilled arrow indicates the preferred direction of air flow.

List of terms indicated in the figures:
- Compact device arranged to reflect images and decontaminate the air (1)
- Ultraviolet radiation emitting system (2)
- Radiation-opaque housing (3)
- Openings in the housing (4)
- Image reflecting system (5)
- Air propulsion system (6)
- Inward-facing areas coated with a material that reflects part of the radiation (7)
- Air particle filtration system (8)
- Air humidity modifying system (9)
- Air temperature modifying system (10)
- Premises illumination system (11)
- Switch-on system actuated by detection of the presence of users in the premises (12)
- Switch-on system actuated by a timer (13)
- Data logging system for environmental parameters (14)
- Data logging system for operating parameters (15)
- User information system arranged to provide users with information regarding operation (16)

### PREFERRED EMBODIMENT OF THE INVENTION

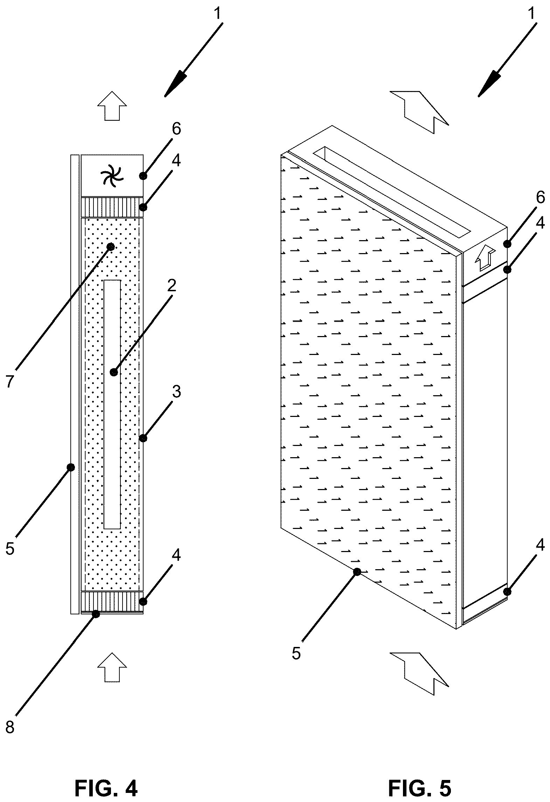

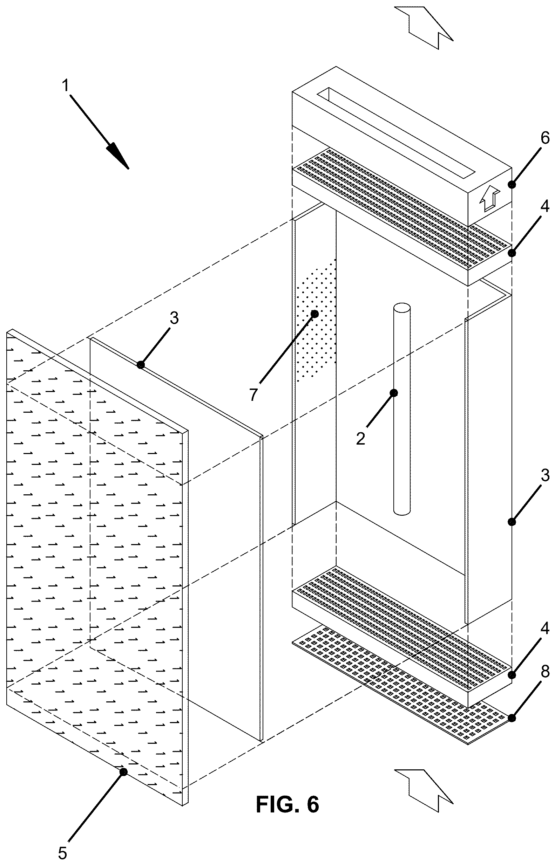

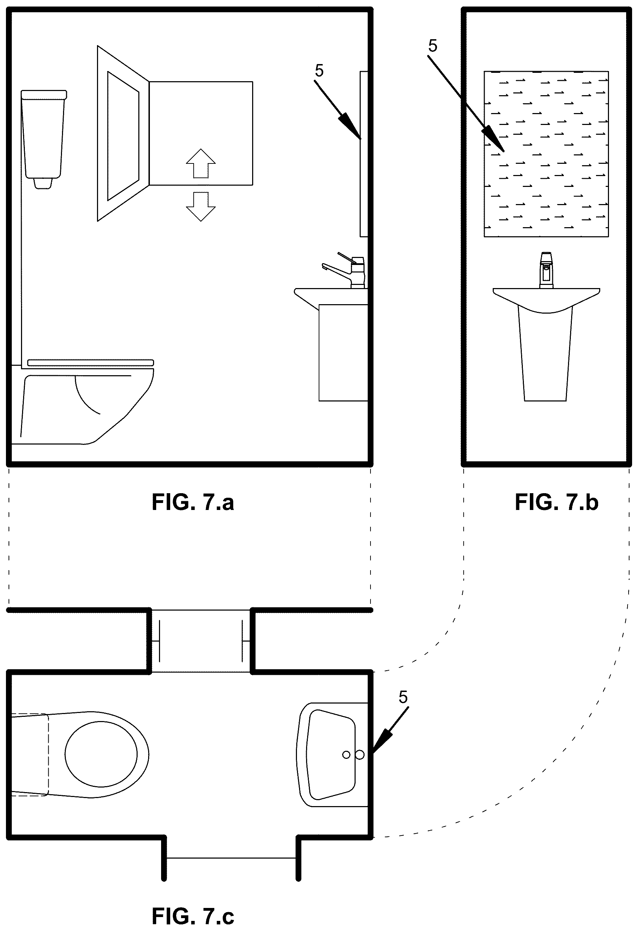

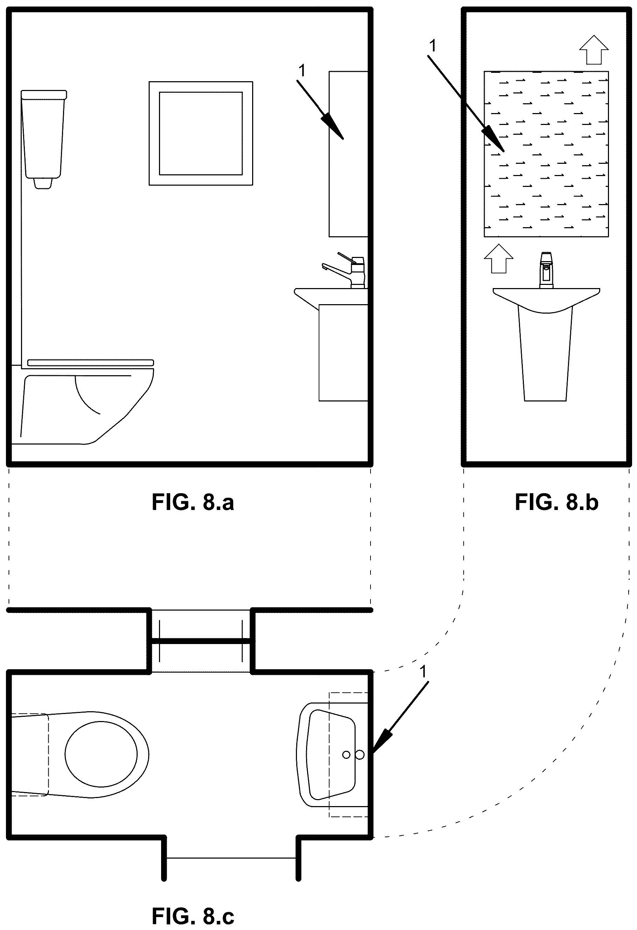

Figures 4 to 8 show a preferred embodiment of a compact device arranged to reflect images and decontaminate the air (1).

It is intended to be installed in the lavatory of a public-use building, which is ventilated by windows that, in order to avoid odours, are always open, so that, depending on the time of year, the energy consumption for air conditioning is high but the temperature is not comfortable. The design dimensions of the lavatory are old and smaller than those prescribed by current regulations; consequently, it is not possible to reduce any of them.

The premises include a mirror above the washbasin with dimensions of 60×80 cm, and require a ventilation flow rate of 10 litres/second, which may be replaced by treatment of a similar air volume. Likewise, and since the building is of a certain age, it is intended to prevent the proliferation of the toxic black mould "Stachybotrys chartarum", for which doses of 500 J/m² are proposed, with aerosol exposure times of 10 seconds. Consequently, the chamber size is 100 litres, which, for the dimensions of the existing mirror of 60×80 cm, implies a thickness of 21 cm, which does not pose any problem above the washbasin.

The device is constructed by means of a radiation-opaque housing (3) made of steel, in the form of a rectangular prism whose base has the same dimensions as the existing mirror and a thickness of 21 cm, and which comprises inward-facing areas coated with a material that reflects part of the radiation (7), namely aluminium paint, an ultraviolet radiation emitting system (2) being arranged inside, said system comprising a discharge lamp capable of providing the target dose within the intended time. One of the larger faces is positioned where the mirror was, and on the opposite larger face there is arranged an image reflecting system (5) formed by a conventional mirror, understood as one formed by transparent glass silvered on its rear face over its entire extent.

The upper and lower sides are left as openings in the housing (4), and on each of them there is arranged, as a device that allows air to pass but blocks radiation, for example a plurality of perforated steel plates with non-aligned orifices; a granular bed is better, but is protected by the granted Patent Publication No. ES2907199 (2024).

The air inlet is at the lower part and is protected by an air particle filtration system (8) formed by an orthogonal steel mesh with a 2 mm pitch.

The air outlet is at the upper part and, above it, an air propulsion system (6) is arranged by means of a cross-flow fan (6) which draws air with sufficient power to overcome the pressure losses of the components described and to provide the required flow rate of 10 litres/second.

Given the industrial nature of all the components indicated above, it is evident that the proposed invention is susceptible of industrial application; it may be carried out by a person skilled in the art and applied in different models providing particular performance capabilities.

## Claims

1. Compact device arranged to reflect images and decontaminate the air (1), **characterised in that** it comprises an ultraviolet radiation emitting system (2), a radiation-opaque housing (3) surrounding said emitting system so as to form a radiation chamber, one or more openings in the housing (4) for air inlet to and air outlet from the radiation chamber, each opening comprising a device arranged to allow the passage of air and prevent the escape of radiation, wherein the housing surrounding the radiation chamber has a flattened shape such that its height is smaller than the length or width of its bases, an image reflecting system (5) mounted on one of said bases and arranged to break upon impact with an amount of energy that does not cause breakage of the housing, and an air propulsion system (6) arranged to move air of the premises through the radiation chamber.

2. Compact device arranged to reflect images and decontaminate the air (1) according to claim 1, wherein the ultraviolet radiation emitting system (2) comprises two or more separate sources spaced apart at distances selected to improve uniformity of the radiation flux.

3. Compact device arranged to reflect images and decontaminate the air (1) according to claim 1 or 2, wherein the radiation-opaque housing (3) comprises inward-facing areas coated with a material that reflects part of the radiation (7).

4. Compact device arranged to reflect images and decontaminate the air (1) according to any one of claims 1 to 3, comprising an air particle filtration system (8).

5. Compact device arranged to reflect images and decontaminate the air (1) according to any one of claims 1 to 4, comprising an air humidity modifying system (9).

6. Compact device arranged to reflect images and decontaminate the air (1) according to any one of claims 1 to 5, comprising an air temperature modifying system (10).

7. Compact device arranged to reflect images and decontaminate the air (1) according to any one of claims 1 to 6, comprising a premises illumination system (11).

8. Compact device arranged to reflect images and decontaminate the air (1) according to any one of claims 1 to 7, comprising a switch-on system actuated by detection of the presence of users in the premises (12).

9. Compact device arranged to reflect images and decontaminate the air (1) according to any one of claims 1 to 8, comprising a switch-on system actuated by a timer (13).

10. Compact device arranged to reflect images and decontaminate the air (1) according to any one of claims 1 to 9, comprising a data logging system for environmental parameters (14).

11. Compact device arranged to reflect images and decontaminate the air (1) according to any one of claims 1 to 10, comprising a data logging system for operating parameters (15).

12. Compact device arranged to reflect images and decontaminate the air (1) according to any one of claims 1 to 11, comprising a user information system arranged to provide users with information regarding operation (16).

13. Compact device arranged to reflect images and decontaminate the air (1) according to any one of claims 1 to 12, wherein the image reflecting system (5) is a conventional mirror, understood as one made of transparent glass silvered on its rear face.
